# DEMANDE DE BREVET EUROPEEN

(11) **EP 0 601 946 A1**
(43) Date de publication de la demande: **15.06.1994**
(21) Numéro de dépôt: 93430016.1
(22) Date de dépôt: 23.11.1993
(51) Int. Cl.: A61N 5/06, G01J 3/12, F21V 9/10

(54) **Dispositif de production d'un faisceau lumineux monochromatique**

(30) Priorité: 04.12.1992 FR 9214895
(71) Demandeur: Chazallet, Frédéric, F-13003 Marseille (FR)
(72) Inventeur: Chazallet, Frédéric, F-13003 Marseille (FR)
(74) Mandataire: Somnier, Jean-Louis

(57) **Abrégé**

La présente invention est relative à un dispositif de production d'un faisceau lumineux monochromatique.

Le dispositif comporte un boîtier (2) portable comportant une source (1) lumineuse, lequel boîtier comporte un filtre optique de faible largeur de bande et de longueur d'onde centrale variable et comporte de moyens pour modifier cette longueur d'onde ; le dispositif comporte une poignée (5) de manipulation et des moyens (8) de transport du faisceau lumineux entre le boîtier et la poignée.

Le domaine technique de l'invention est celui des dispositifs de traitement thérapeutique du corps humain ou animal à l'aide de rayons lumineux.

## Description

La présente invention est relative à un dispositif de production d'un faisceau lumineux monochromatique essentiellement destiné à la chromothérapie, à l'actinothérapie, à la chromatothérapie ou à la chromopuncture.

Le domaine technique de l'invention est celui des dispositifs de traitement thérapeutiques du corps humain ou animal à l'aide de rayons lumineux situés notamment dans le domaine de la lumière visible.

Il est connu d'exposer tout ou partie du corps humain ou animal à un rayonnement de lumière visible notamment, dans un but thérapeutique, notamment pour le traitement de maladies cutanées ou bien pour le traitement d'états de fatigue ainsi que diverses infections et traumatismes.

La demande de brevet FR2 668 068 (LERNER) par exemple décrit un procédé d'exposition d'un corps à des rayonnements lumineux monochromes et un dispositif de mise en oeuvre de ce procédé ; selon le procédé décrit dans ce document, on illumine le corps à traiter selon des séquences d'éclairement monochromes successifs délivrés par des sources individuelles lumineuses monochromatiques qui sont mises en service séquentiellement afin de décrire le spectre lumineux.

Le dispositif de mise en oeuvre décrit dans ce document comporte un panneau éclairant supérieur et une couchette inférieure constituée par un lit solaire transparent situé au dessus de tubes de rayonnements monochromes identiques à ceux du panneau supérieur.

La demande de brevet EP-A-488 333 déposée le 28/11/91 décrit un dispositif d'excitation chromatique de quartz à des buts thérapeutiques, qui comporte une source lumineuse d'intensité et de longueur d'onde variables controlée par un ordinateur et équipée de filtres ; l'applicateur comportant des cristauux de quartz est relié au générateur par des fibres optiques.

La présente invention a pour objet de procurer un dispositif de production d'un faisceau lumineux monochromatique destiné notamment à l'actinothérapie qui permette un traitement sélectif, c'est-à-dire qui permette de traiter sélectivement (uniquement) une zone déterminée du corps humain ou animal de relativement faibles dimensions, et qui permette également de traiter ladite zone du corps humain à l'aide d'un rayonnement sensiblement monochromatique, c'est-à-dire un rayonnement dont l'essentiel de l'énergie est concentré dans une bande de fréquences ou de longueurs d'ondes étroite.

L'invention consiste également à procurer un tel dispositif qui soit facile à manipuler par un praticien et qui soit susceptible d'être fabriqué à moindre coût.

La solution au problème posé consiste à procurer un dispositif de production d'un faisceau lumineux sensiblement monochromatique, essentiellement situé dans le domaine de la lumière visible et/ou du proche infrarouge et/ou du proche ultra violet, lequel dispositif est destiné particulièrement à la chromothérapie et/ou à l'actinothérapie ; le dispositif comporte un boîtier portable comportant (contenant) une source lumineuse (de lumière non cohérente à spectre large, telle qu'une lampe à incandescence utilisant au moins un gaz halogène, par exemple d'une puissance de l'ordre de 50 watts), lequel boîtier comporte (contient) des moyens de filtre optique passe bande, de faible largeur de bande, et de longueur d'onde centrale (ou fréquence correspondante) de préférence continûment variable, et comporte (contient) des moyens pour modifier de préférence, continûment, ladite longueur d'onde centrale, lequel dispositif comporte en outre une poignée (ou manchon) de manipulation permettant de diriger ledit faisceaux lumineux sur une zone cutanée du corps humain à traiter et permettant de positionner ladite poignée à une distance prédéterminée de ladite zone cutanée à traiter ; ledit dispositif comporte des moyens de transport dudit faisceau lumineux produit par ladite source lumineuse entre ledit boîtier et ladite poignée.

Avantageusement, ledit moyen de filtre optique passe bande de longueur d'onde centrale variable comporte un filtre interférentiel hétérogène (c'est-à-dire dont la longueur d'onde centrale n'est pas constante sur la surface utile dudit filtre) et mobile.

Avantageusement, ledit filtre interférentiel a une forme de bande sensiblement étroite (dont le rapport de la longueur L1 à la largeur L2 est au moins égal à 5 par exemple), sensiblement rectiligne, qui peut être cintrée selon un rayon R7 (par exemple de l'ordre de 50 mm) ; ladite bande comporte des dépôts successifs de couches minces alternées d'au moins deux matériaux prédéterminés (tels que par exemple de la cryolithe et du sulfure de zinc ZnS),lesquels dépôts peuvent être effectués sur un support ou plaque (dans laquelle ladite bande est ensuite découpée) en matière plastique transparente et souple telle que du polycarbonate, du polymétacrilate de métyl (PMMA), du triacétate..., ladite bande est montée sur un support cylindrique d'axe XX1 susceptible d'être entrainé en rotation selon ledit axe par un moteur, de préférence un moteur pas à pas intégré audit boîtier.

Avantageusement, ledit boîtier comporte (contient) un filtre infrarouge parfois appelé filtre de blocage infrarouge ou filtre d'absorbsion infrarouge (passe bas en terme de longueur d'onde, c'est-à-dire absorbant des longueurs d'onde supérieures ou égales à 1 micromètre) intercalé entre ladite source lumineuse et ledit filtre interférentiel.

La solution au problème posé consiste en d'autres termes à procurer un dispositif d'actinothérapie produisant un faisceau lumineux sensiblement monochromatique, comportant un boîtier portable comportant une source lumineuse, lequel boîtier comporte un filtre interférentiel hétérogène mobile monté sur un support entraîné par un moteur, lequel dispositif comporte en outre une poignée de manipulation et des moyens de transport dudit faisceaux lumineux entre ledit boîtier et ladite poignée, lequel boîtier comporte un filtre infrarouge intercallé entre ladite source lumineuse et ledit filtre interférentiel.

Avantageusement un filtre passebande rouge est monté en série avec ledit filtre interférentiel et transmet des radiations de longueur d'onde supérieures à 650 nanomètres environ et absorbe les longueurs d'ondes inférieures à 600 nanomètres environ, lequel dispositif comporte en outre un filtre passebande bleu monté en série avec ledit filtre interférentiel, transmettant des longueurs d'ondes comprises entre 425 et 500 nanomètres environ et absorbe les longueurs d'ondes comprises entre 550 et 750 nanomètres environ, lesquels filtres passebande rouge et bleu sont du type filtre gélatine, sont montés et de préférence collés sur ledit support recevant ledit filtre interférentiel, lesquels filtres passebande rouge et bleu s'étendent respectivement dans une zone dudit support situé en regard d'une partie respective dudit filtre interférentiel dont les longueurs d'ondes centrales de bandes passantes sont supérieures à 650 nanomètre environ, respectivement inférieures à 500 nanomètres environ.

Avantageusement ledit filtre infra rouge est essentiellement constitué par une plaque de verre sensiblement épaisse, par exemple d'épaisseur voisine de 1 à 5 mm, montée sensiblement perpendiculairement à l'axe du faisceau lumineux produit par ladite source lumineuse, dont le taux de transmission intrinsèque est de préférence inférieur ou égal à 2 % dans toute la plage des longueurs d'ondes situées entre 1000 et 3000 nanomètres.

Avantageusement, ledit dispositif comporte (intégré audit boîtier) une unité centrale à microprocesseur, un détecteur de position dudit filtre et/ou dudit support de filtre, des moyens d'enregistrement (tels qu'une mémoire au moins) d'au moins deux couples de données d'étalonnage, lesquels couples de données d'étalonnage comportent au moins un premier couple de données d'étalonnage comportant une première donnée de position dudit filtre et au moins une première longueur d'onde centrale correspondante (qui peut être mesurée par un dispositif d'étalonnage), et comportent au moins un deuxième couple de données d'étalonnage comportant une deuxième donnée de position dudit filtre et une deuxième longueur d'onde centrale correspondante (qui peut être mesurée par ledit dispositif d'étalonnage), lesquels couples de données d'étalonnage peuvent être enregistrés dans ladite mémoire et protégés contre l'effacement.

Avantageusement, ledit dispositif comporte des moyens d'enregistrement de plusieurs données de position dudit filtre correspondant à des longueurs d'ondes centrales que l'utilisateur désire mémoriser.

Avantageusement, ledit dispositif comporte des moyens d'enregistrement d'une pluralité de couples de données, chacun desdits couples de données comportant une première donnée relative à une durée d'éclairement et une deuxième donnée correspondant à une longueur d'onde centrale correspondante, prédéterminée.

Avantageusement, ledit dispositif comporte des moyens de calcul par interpolation (par exemple linéaire) entre lesdites données d'étalonnage de sorte que lorsqu'on souhaite que ledit dispositif produise ledit faisceau lumineux monochromatique autour d'une longueur d'onde centrale déterminée, on introduit une valeur représentative de ladite longueur d'onde centrale déterminée ou souhaitée, et lesdits moyens de calcul par interpolation déterminent une position, par exemple une position angulaire dudit filtre et/ou dudit support de filtre, qui permettra d'obtenir ladite longueur d'onde centrale désirée par interpolation entre des données d'étalonnage préalablement enregistrées.

Avantageusement, ladite bande passante à moins trois décibel desdits moyens de filtre est de l'ordre de 10 nanomètres, dans une bande de longueur d'onde par exemple comprise entre 400 (ou 450) et 650 (ou 700) nanomètres, et ledit dispositif permet d'obtenir une résolution (ou précision ou écart entre une longueur d'onde centrale et une longueur d'onde centrale obtenue) qui est de l'ordre de dix nanomètres.

Avantageusement, ledit boîtier a une forme générale pyramidale, et ledit dispositif comporte une interface numérique de communication avec un calculateur externe, et comporte un clavier permettant l'introduction de données, notamment de données relative à la longueur d'onde centrale et à la durée d'éclairement désirées, et comporte un afficheur permettant de visualiser notamment la longueur d'onde dudit faisceau lumineux monochromatique correspondant à la position courante dudit filtre interférentiel mobile.

Le dispositif de production d'un faisceau lumineux monochromatique selon l'invention comporte de nombreux avantages ; le dispositif selon l'invention permet un traitement sélectif d'une zone déterminée du corps humain ou animal à une ou plusieurs longueurs d'ondes centrales, et plus précisément selon des séquences prédéterminées d'illumination par un faisceau lumineux dont l'essentiel de l'énergie est concentré dans des bandes spectrales ou bandes de longueurs d'ondes étroites par exemple de l'ordre 10 nanomètres de largeur.

L'invention permet de procurer de tels dispositifs qui peuvent être fabriqués à moindre coût, qui peuvent être portables ou portatifs et qui, grâce notamment à la présence de ladite poignée comportant une optique de focalisation du faisceau lumineux de sortie, laquelle poignée est raccordée audit boîtier contenant ladite source lumineuse par un dispositif de raccordement tel qu'un câble à fibres optiques, permet une manipulation aisée par le praticien de ladite poignée et facilite ainsi le traitement sélectif d'une zone prédéterminée du corps humain à traiter ; les dispositifs de production d'un faisceau lumineux monochromatique selon l'invention, sont également faciles d'utilisation grâce à leurs moyens d'interface avec d'une part l'utilisateur, lui permettant de choisir simplement les longueurs d'ondes et/ou les séquences d'illumination voulues, laquelle facilité d'utilisation peut être encore augmentée par le raccordement d'un dispositif selon l'invention à un calculateur ou ordinateur permettant d'enregistrer de façon permanente des données de traitement spécifiques d'une maladie et/ou d'un patient déterminé ; dans ce cas, après sélection de la pathologie à traiter, le dispositif peut effectuer automatiquement, sous le contrôle dudit ordinateur, une séquence d'éclairements de durées et de longueurs d'onde centrale prédéterminées (préalablement enregistrées dans une mémoire de l'ordinateur) correspondant à ladite pathologie.

Grâce à la structure particulière d'un dispositif selon l'invention, on dispose d'un ensemble mécanique léger comportant ledit filtre interférentiel mobile monté sur son support, qui peut être déplacé précisément et rapidement par un moteur de très faible puissance ; grâce à la présence et aux caractéristiques particulières du filtre infrarouge, et avec l'aide d'un ventilateur, on peut obtenir un ensemble mécanique compact qui supporte l'élévation de température due au rayonnement infrarouge de la source lumineuse, grâce à l'évacuation de chaleur opérée par le flux d'air entrainé par le ventilateur.

Grâce au filtre interférentiel hétérogène mobile, on peut faire varier continuement la longueur d'onde désirée du faisceau lumineux.

Les nombreux avantages procurés par l'invention, seront mieux compris au travers de la description suivante qui se réfère aux dessins annexés, qui illustrent sans aucun caractère limitatif, un mode particulier de réalisation de dispositif selon l'invention.

La figure 1 illustre en vue en plan les principaux constituants d'un dispositif d'émission d'un faisceau monochromatique de longueur d'onde variable d'un dispositif selon l'invention ; la figure 1 est une vue selon I-I de la figure 2.

La figure 2 illustre en coupe verticale longitudinale un mode de réalisation d'un boîtier d'un dispositif selon l'invention.

La figure 3 est une vue selon III-III de la figure 2 et illustre en vue de dessus un boîtier d'un dispositif selon l'invention.

La figure 4 illustre schématiquement sous forme de synoptique les principaux composants des moyens de contrôle et de commande d'un dispositif selon l'invention.

La figure 5 illustre schématiquement les principaux constituants d'un dispositif selon l'invention et leur mode de mise en oeuvre.

La figure 6 illustre un mode préférentiel de réalisation d'un filtre interférentiel ayant une forme de bande et utilisé dans un dispositif selon l'invention.

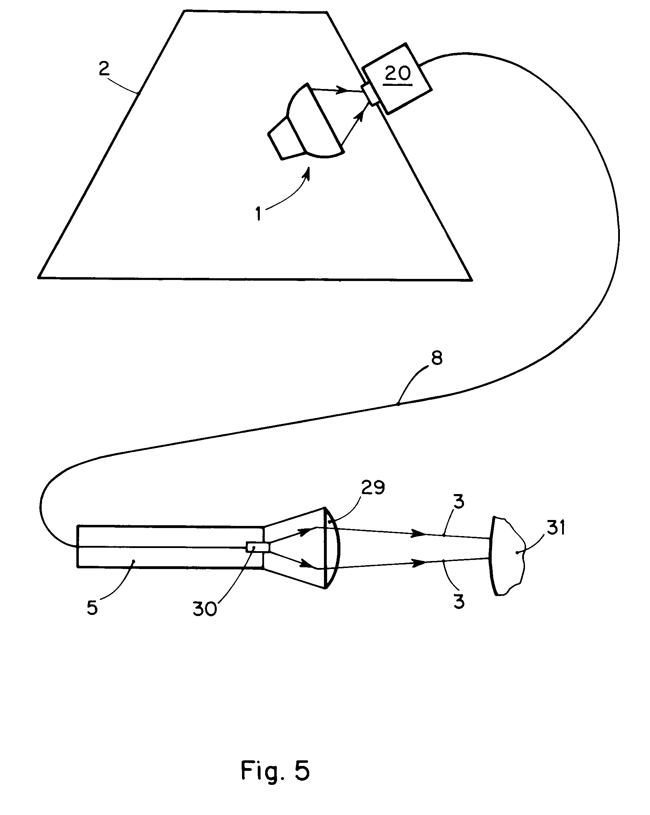

Par référence à la figure 5, on voit qu'un dispositif selon l'invention comporte essentiellement d'une part un boîtier 2 qui peut avoir de préférence la forme d'une pyramide tronquée, lequel boîtier 2 comporte une source lumineuse 1 émettant un faisceau lumineux qui peut être dirigé vers la zone 31 du corps du patient à traiter par l'intermédiaire d'un câble 8 à fibres optiques raccordé audit boîtier 2 par un connecteur 20, lequel câble permet la transmission dudit faisceau lumineux émis par ladite source lumineuse 1 jusqu'à une poignée 5 munie d'un connecteur de sortie 30 raccordé audit câble de fibres optiques 8, lequel connecteur de sortie 30 permet la production en sortie dudit câble de fibres optiques, d'un faisceau 3 qui peut de préférence passer dans une lentille 29 de focalisation dudit faisceau 3 vers ladite zone prédéterminée du corps du patient à traiter.

Grâce à la configuration générale du dispositif selon l'invention, ladite poignée peut permettre de focaliser ledit faisceau 3 lumineux monochromatique obtenu en sortie du dispositif à une distance, mesurée entre ladite optique de sortie 29 de ladite poignée et ladite zone à traiter, qui peut être inférieure à un mètre de manière à produire un spot ou tâche lumineuse sur ladite zone à traiter ayant une taille de quelques centimètres ou quelques dizaines de centimètres ; le maniement de la poignée est uniquement limité par la longueur du câble 8 à fibres optiques qui est souple et léger et permet ainsi de traiter sélectivement par le déplacement de ladite poignée relativement audit boîtier la zone du corps à traiter en laissant ledit boîtier dans une position fixe.

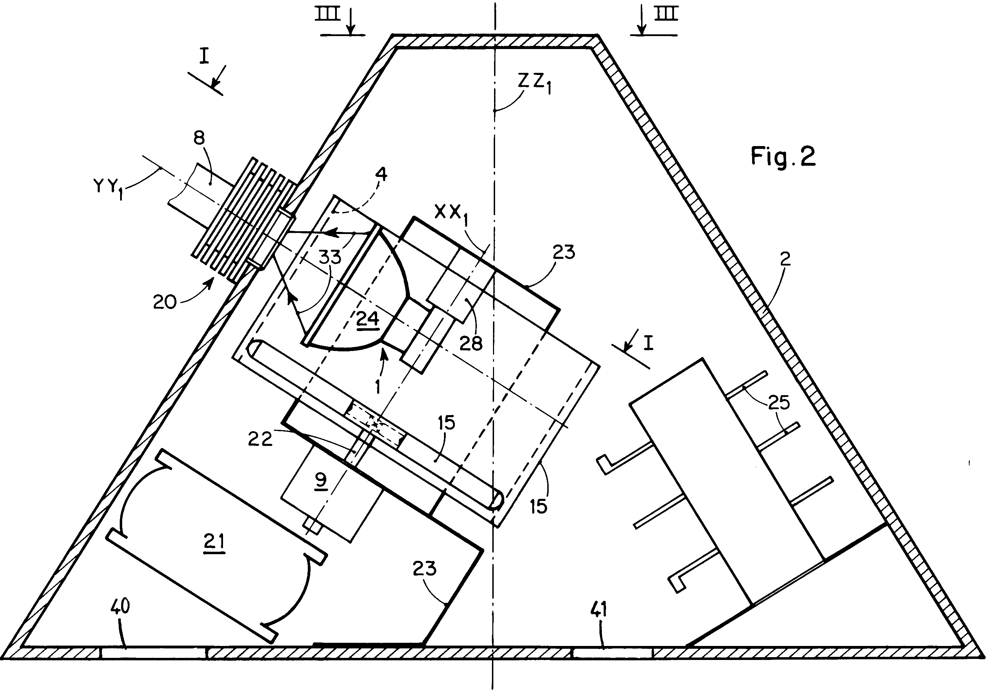

Par référence à la figure 2 on voit que ledit boîtier 2 comporte ladite source lumineuse 1 qui peut être par exemple une lampe halogène munie d'un réflecteur dichroïque 24 et qui est montée de manière fixe à l'intérieur dudit boîtier par l'intermédiaire d'un support 28 lui même fixé à un support 23 fixé audit boîtier 2.

Ledit support 23 peut également supporter un moteur 9 d'entraînement en rotation selon un axe XX1 d'un support 15 de filtre 4, de préférence d'un filtre interférentiel ; ledit support 15 a de préférence une forme générale de tambour cylindrique ouvert à son extrémité supérieure de sorte que ladite source lumineuse 1 peut être contenue dans l'espace située à l'intérieur dudit tambour cylindrique et peut émettre un faisceau lumineux initial 33 d'axe optique YY1 sensiblement perpendiculaire audit axe XX1 de rotation dudit tambour support de filtre ; ledit faisceau initial 33 peut se propager à travers ledit filtre interférentiel 4 pour aboutir audit connecteur optique 20 situé à une première extrémité dudit câble 8 à fibres optiques reliant ledit boîtier 2 à ladite poignée de manipulation (non représentée sur cette figure).

On voit également sur cette figure que ledit boîtier intègre des cartes électroniques 25 recevant les moyens de contrôle et de commande du dispositif et peut également intégrer un ventilateur 21 de refroidissement de ladite lampe et desdits filtres et desdites cartes électroniques, qui provoque une circulation d'air pénétrant par une ouverture 40 et sortant par une ouverture 41, lesquelles ouvertures sont prévues dans la paroi dudit boitier 2.

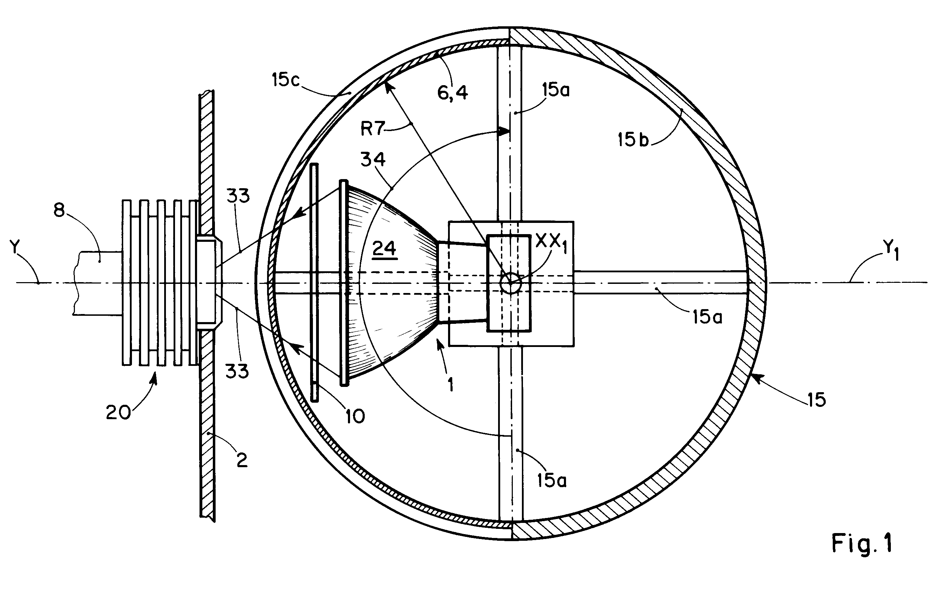

Par référence à la figure 1 on voit que dans un mode préférentiel de réalisation ledit filtre interférentiel 4 a une forme de bande 6 cintrée selon un rayon R7 qui peut être de l'ordre d'une dizaine de centimètres par exemple, laquelle bande s'étend selon une demi circonférence dans une partie 15C dudit support mobile 15 en forme de tambour ; ladite partie 15C constitue une fenêtre prévue dans la périphérie dudit tambour de sorte que ledit faisceau lumineux 33 issue de ladite source lumineuse 1 peut traverser ledit filtre interférentiel, quelle que soit la position angulaire prise par ledit support 15 du filtre lorsque ledit support 15 mu par ledit moteur (non représenté) tourne selon ledit axe de rotation XX1, entre deux positions extrêmes séparées par un angle 34 (de préférence compris entre 90 et 270 degrés) de déviation ou rotation maximale dudit support 15.

Avantageusement, un filtre infrarouge 10 est intercalé entre ladite source lumineuse 1 munie dudit réflecteur 24 (qui est montée fixe par rapport audit boîtier 2 partiellement représenté) et ledit filtre 4 mobile monté sur ledit support mobile 15, de manière à intercepter une partie du flux lumineux situé dans le domaine infrarouge et de manière à éviter ainsi l'échauffement dudit filtre interférentiel 4.

On voit également sur cette figure que ledit connecteur optique 20 monté sur la paroi 2 dudit boîtier et situé à une extrémité dudit câble à fibres optiques 8 est prévu pour acheminer ledit faisceau 33 ayant traversé ledit filtre interférentiel 4.

On voit également sur cette figure que l'axe YY1 optique général du dispositif est situé dans plan perpendiculaire audit axe XX1 de rotation dudit filtre 4 et/ou dudit support 15 de filtre, lequel support 15 de filtre a une forme générale tubulaire à paroi mince et est doté de raidisseurs diamétraux 15A lui permettant sa fixation sur l'arbre dudit moteur (non représenté).

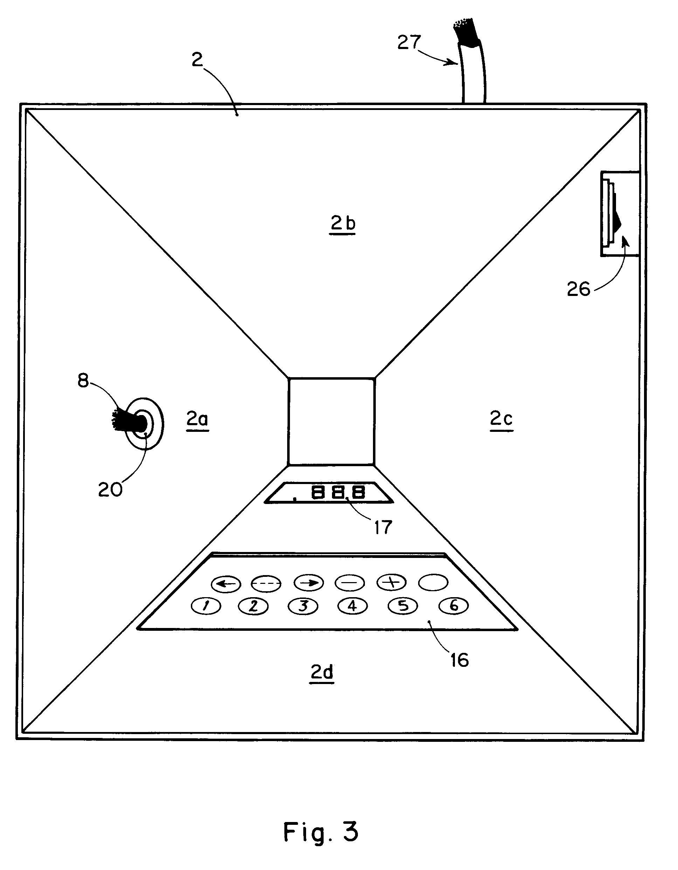

Par référence à la figure 3 on voit que ledit boîtier 2 a une forme de pyramide tronquée munie de quatre faces latérales 2a, 2b, 2c, 2d ; ladite face 2a reçoit ledit connecteur 20 situé à l'extrémité dudit câble à fibres optiques 8 ; ladite face 2c reçoit un interrupteur 26 de commande de marche ou d'arrêt dudit dispositif qui peut être alimenté par un câble d'alimentation électrique 27.

Ladite face 2d peut recevoir un afficheur 17 permettant de visualiser par exemple la longueur d'onde centrale courante c'est-à-dire la longueur d'onde correspondant à la position courante ou actuelle dudit support de filtre (repère 15 des figures 1 et 2) ; ladite face 2d comporte également un clavier 16 permettant à l'utilisateur ou au praticien de sélectionner des longueurs d'ondes qui ont pu être préalablement enregistrées et/ou de commander les différents paramètres de fonctionnement de l'appareil.

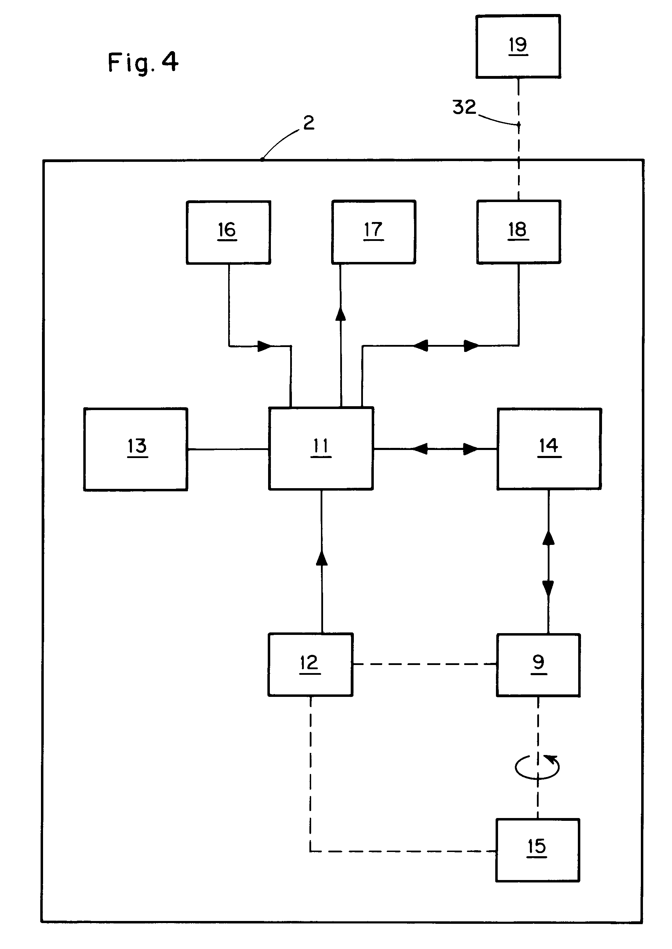

Par référence à la figure 4 on voit que lesdits moyens de commande et de contrôle du dispositif (qui peuvent faire l'objet desdites cartes électroniques repèrées 25 sur la figure 2) peuvent comporter essentiellement une unité de commande 11 par exemple à microprocesseur, relié à une mémoire 13 dans laquelle peuvent être enregistrées lesdites valeurs d'étalonnage du dispositif et lesdites valeurs prédéterminées et mémorisées de position angulaire dudit support de filtre et desdites longueurs d'ondes centrales correspondantes ; ladite unité de commande 11 à microprocesseur peut être relié à un module 14 d'interface et de commande de puissance dudit moteur 9 tel qu'un moteur pas à pas qui peut entraîner en rotation ledit support 15 de filtre interférentiel ; un détecteur 12 de la position dudit arbre dudit moteur et/ou dudit support 15 de filtre, est relié à ladite unité de commande 11, lui permettant de connaître à chaque instant la position dudit support et d'en déduire, par rapport auxdites valeurs d'étalonnage, la longueur d'onde centrale courante correspondant à ladite position courante dudit support de filtre .

Les moyens de contrôle de position peuvent comprendre ledit détecteur 12 d'une position d'origine (ou "zéro") par exemple par un capteur optique ou mécanique, et des moyens de comptage du nombre de pas effectués par ledit moteur.

Ladite unité de commande 11 est également reliée audit clavier 16 et audit afficheur 17 et peut être avantageusement reliée par l'intermédiaire d'une interface numérique 18 à un calculateur 19 extérieur par l'intermédiaire de moyens de liaison 32 ; l'ensemble des composants représentés à la figure 4 (à l'exception dudit calculateur) étant intégré audit boîtier 2 du dispositif selon l'invention.

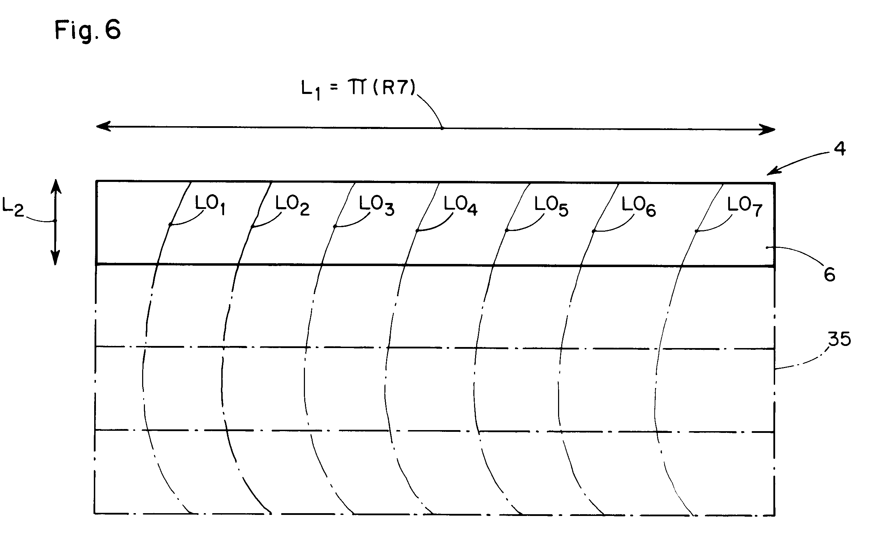

Par référence à la figure 6, on voit que dans un mode préférentiel de réalisation ledit filtre interférentiel 4 se présente sous la forme d'une bande 6 allongée rectiligne de relativement faible largeur L2 par rapport à sa longueur L1 qui est sensiblement égale à la portion de circonférence( par exemple la demi circonférence) correspondant audit rayon repéré R7 à la figure 1 de manière à pouvoir être cintré sur ledit support de filtre.

Ledit filtre interférentiel 4 sous forme de bande 6, peut être obtenu par découpe dans une plaque 35 sur laquelle a été opéré le dépôt en couches minces d'au moins deux matériaux permettant la réalisation dudit filtre interférentiel, lequel dépôt, du fait de la position relative de ladite plaque par rapport aux sources de sublimation desdits matériaux provoque des variations spaciales (dans le plan de ladite plaque 35) desdits dépôts de couches minces, lesquelles variations de dépôts provoquent des variations spaciales (ou gradients) dans les longueurs d'ondes centrales ; comme représenté à la figure 6, dans une zone de ladite bande 6 (formant support dudit filtre interférentiel 4) située à gauche sur la figure 6, ladite zone peut correspondre à une longueur d'onde centrale de la bande passante dudit filtre correspondant à une première valeur L01 ; lorsqu'on se déplace sur ladite bande 6 de gauche à droite par référence à la figure 6, on peut ainsi déterminer des zones ou segments correspondant à d'autres longueurs d'ondes centrales de bande passante dudit filtre interférentiel, qui sont respectivement repérés L02, L03, L04, L05, L06, L07, lesquelles valeurs sont sensiblement continuement soit croissantes soit décroissantes ; ainsi lorsque ledit faisceau lumineux issu de ladite source lumineuse traverse ladite bande 6 formant support du filtre interférentiel 4 dans une zone proche de la zone repéré L01, le faisceau de sortie ayant traversé ledit filtre est sensiblement centré sur une longueur d'onde égale à ladite longueur d'onde L01, qui peut être mesurée lors d'un étalonnage de l'appareil en usine, afin de palier à la difficulté de répétabilité et aux variations qui peuvent être provoquées du fait de la géométrie des dépôts de couches minces sur les différentes bandes 6 découpées dans ladite plaque 35.

Une variation sensiblement continue de la longueur d'onde centrale dudit faisceau lumineux ayant traversé ledit filtre interférentiel, peut ainsi être obtenue par une rotation (un déplacement) sensiblement continue dudit filtre et/ou dudit support de filtre.

## Revendications

1. Dispositif d'actinothérapie produisant un faisceau lumineux (3) sensiblement monochromatique, comportant un boîtier (2) portable comportant une source (1) lumineuse, lequel boîtier comporte un filtre (4) interférentiel hétérogène mobile monté sur un support (15) entraîné par un moteur (9), lequel dispositif comporte en outre une poignée (5) de manipulation et des moyens (8) de transport dudit faisceaux lumineux entre ledit boîtier et ladite poignée, lequel boîtier comporte un filtre infrarouge (10) intercalé entre ladite source lumineuse et ledit filtre interférentiel.

2. Dispositif selon la revendication 1 comportant en outre un filtre passebande rouge monté en série avec ledit filtre interférentiel, transmettant des radiations de longueur d'onde supérieures à 650 nanomètres, lequel dispositif comporte en outre un filtre passebande bleu monté en série avec ledit filtre interférentiel, transmettant des longueurs d'ondes comprises entre 425 et 500 nanomètres, lesquels filtres passebande rouge et bleu sont du type filtre gélatine, sont montés et de préférence collés sur ledit support (15) recevant ledit filtre interférentiel, lesquels filtres passebande rouge et bleu s'étendent respectivement dans une zone dudit support située en regard d'une partie respective dudit filtre interférentiel dont les longueurs d'ondes centrales de bandes passantes sont supérieures à 650 nanomètre, respectivement inférieures à 500 nanomètres.

3. Dispositif selon l'une quelconque des revendications 1 à 2 dans lequel ledit filtre infra rouge est essentiellement constitué par une plaque de verre sensiblement épaisse, par exemple d'épaisseur voisine de 1 à 5 mm, montée sensiblement perpendiculairement à l'axe du faisceau lumineux produit par ladite source lumineuse, dont le taux de transmission intrinsèque est de préférence inférieur ou égal à 2 % dans toute la plage des longueurs d'ondes situées entre 1000 et 3000 nanomètres.

4. Dispositif selon l'un quelconque des revendications 1 à 3 comportant une interface numérique de communication avec un calculateur externe, un clavier permettant l'introduction de données relatives à la longueur d'onde centrale et à la durée d'éclairement désirée, un afficheur (17) permettant de visualiser la longueur d'onde dudit faisceau lumineux.

5. Dispositif selon l'une quelconque des revendications 1 à 4 dans lequel ledit filtre interférentiel a une forme de bande (6) étroite, sensiblement rectiligne, qui peut être cintrée selon un rayon (R7) laquelle bande est montée sur un support (15) cylindrique d'axe XX1 susceptible d'être en rotation selon ledit axe par un moteur (9) pas à pas intégré audit boîtier.

6. Dispositif selon l'une quelconque des revendications 1 à 5 comportant une unité centrale (11) à microprocesseur, un détecteur (12) de position dudit filtre et/ou dudit support de filtre, des moyens d'enregistrement d'au moins deux couples de données d'étalonnage, un premier couple de données d'étalonnage comportant une première donnée de position dudit filtre et une première donnée de longueur d'onde centrale correspondante un deuxième couple de données d'étalonnage comportant une deuxième donnée de position dudit filtre et une deuxième longueur d'onde centrale correspondante.

7. Dispositif selon l'une quelconque des revendications 1 à 6 comportant des moyens (13) d'enregistrement de plusieurs données de position dudit filtre correspondant à des longueurs d'ondes centrales que l'utilisateur désire mémoriser.

8. Dispositif selon l'une quelconque des revendications 1 à 7 comportant des moyens d'enregistrement (13) d'une pluralité de couples de données, chacun desdits couples de données comportant une donnée relative à une durée d'éclairement et une deuxième donnée correspondant à une longueur d'onde centrale correspondante prédéterminée.

9. Dispositif selon l'une quelconque des revendications 1 à 8 comportant des moyens de calcul par interpolation linéaire entre lesdites données d'étalonnage de sorte que lorsqu'on souhaite que ledit dispositif produise ledit faisceau lumineux monochromatique centré sur une longueur d'onde centrale déterminée, on introduit une valeur représentative de ladite longueur d'onde centrale déterminée, et lesdits moyens de calcul par interpolation déterminent une position dudit filtre et/ou dudit support de filtre qui permettra d'obtenir ladite longueur d'onde centrale désirée, par un calcul d'interpolation entre des données d'étalonnage préalablement enregistrées.

10. Dispositif selon l'une quelconque des revendications 1 à 9 dans lequel ledit boîtier a une forme pyramidale et comporte un ventilateur (21).
